(19) Europäisches Patentamt
European Patent Office
Office européen des brevets

(11) **EP 1 722 839 B1**

(12) **EUROPÄISCHE PATENTSCHRIFT**

(45) Veröffentlichungstag und Bekanntmachung des Hinweises auf die Patenterteilung:
**11.12.2013 Patentblatt 2013/50**

(21) Anmeldenummer: 05715413.0

(22) Anmeldetag: **19.02.2005**

(51) Int Cl.:
*A61M 5/00* *(2006.01)*    *A61M 15/00* *(2006.01)*

(86) Internationale Anmeldenummer:
**PCT/EP2005/001745**

(87) Internationale Veröffentlichungsnummer:
**WO 2005/084731 (15.09.2005 Gazette 2005/37)**

(54) **VORRICHTUNG ZUR ZEITLICHEN DOSIERUNG VON ARZNEISTOFFEN**

DEVICE FOR THE TIMED DOSAGE OF MEDICAMENTS

APPAREIL DE DOSAGE CHRONOMETRIQUE DE MEDICAMENTS

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **04.03.2004 DE 102004010516**

(43) Veröffentlichungstag der Anmeldung:
**22.11.2006 Patentblatt 2006/47**

(73) Patentinhaber: **Bayer Intellectual Property GmbH**
**40789 Monheim (DE)**

(72) Erfinder:
• **WILLMANN, Stefan**
**40593 Düsseldorf (DE)**
• **SCHMITT, Walter**
**41470 Neuss (DE)**
• **LIPPERT, Jörg**
**51375 Leverkusen (DE)**
• **DORN, Ingmar**
**50668 Köln (DE)**

(74) Vertreter: **BIP Patents**
**c/o Bayer Intellectual Property GmbH**
**Creative Campus Monheim**
**Alfred-Nobel-Straße 10**
**40789 Monheim (DE)**

(56) Entgegenhaltungen:
WO-A2-01/83007        DE-U1- 20 005 004
US-A- 4 533 346        US-A- 5 687 208
US-A1- 2002 173 729

**Beschreibung**

Die Erfindung betrifft eine Vorrichtung zur zeitlich gesteuerten Dosierung von Arzneistoffen mittels eines Verfahrens zur Bestimmungen eines entsprechenden Dosierungsprofils und entsprechender Steuerung der Dosiereinrichtung.

Computergesteuerte Infusionspumpen, deren Eingabefunktion mittels eines pharmakokinetischen Modells ermittelt werden, sind nach dem Stand der Technik unter dem Stichwort "TCI" (= Target Controlled Infusion) bekannt und kommerziell erhältlich. Hauptanwendungsgebiet von TCI ist die Steuerung von intravenös verabreichten Narkosemitteln (z. B. Propofol, vermarktet als Diprifusor™ von AstraZeneca (Produktin-formation "Diprifusor™: Target Controlled Infusion (TCI) in anaesthetic practice", AstraZeneca Anaesthesia, New Edition (1998)). Nachteilig bei diesen bekannten Verfahren ist, dass das pharmakokinetische Modell ein an experimentellen Plasma-Daten gefittetes Drei-Kompartiment-Modell ist. Bei einem solchen "Black-Box" Verfahren besteht keine Möglichkeit, individuelle physiologische Faktoren des Patienten im pharmakokinetischen Modell zu berücksichtigen. Im Gegensatz hierzu erlauben physiologie-basierte PK/PD Modelle wie das von Bayer Technology Services GmbH entwickelte PK-Sim® (www.PK-Sim.com; S. Willmann, J. Lippert, M. Sevestre, J. Solodenko, F. Fois, W. Schmitt: "PK-Sim®: a physiologically based pharmacokinetic 'whole-body' model", Biosilico 1, 121-124 (2003)), den Einfluss von individuellen physiologischen und anatomischen Parametern wie z. B. Organgröße und -zusammensetzung, Blutflussraten, etc. auf das zeitliche pharmakokinetische Verhalten von Arzneistoffen zu beschreiben (z.B. DE Anm. Nr. 10160270 und DE Anm. Nr. 10345836). Diese physiologischen und anatomischen Parameter lassen sich wiederum auf einige wenige leicht messbare Größen wie z.B. Körpergewicht und Body Mass Index zurückführen. In DE Anm. Nr. 10345837 wurde darüber hinaus beschrieben, wie auch biochemische und genetische Informationen wie z.B. Expressionsdaten metabolisch aktiver Enzyme oder aktiver Transporter zur Bestimmung einer individuell an den Patienten angepassten Dosierung herangezogen werden können. Diese Systeme sind jedoch reine Simulationsmodelle, die keine direkte Unterstützung einer Anwendung am Patienten oder von klinischen Anwendungen zulassen.

Dokument US 2002/173729 offenbart eine Dosiervorrichtung, die ein Pharmakodynamik und/oder ein Pharmakokinetik-Model mit den gemessenen Patientenparameternkombiniert, um eine zeitliche Steuerung der Dosierung eines Arzneistoffs zu erreichen.

Dokument US 568 7208 offenbart ein Verfahren zur Selektion eines Applikationsprotokolls für Kontrastmittel in einem Patienten in Vorbereitung einer Computertomographie.

Der Erfindung liegt ausgehend vom genannten Stand der Technik die Aufgabe zu Grunde, eine Verbesserte Vorrichtung zu entwickeln, welche eine exakte zeitliche Dosierung eines Medikaments unter Berücksichtigung individueller physiologischer, anatomischer, biochemischer und genetischer Faktoren des Patienten ermöglicht.

Der exakte zeitliche Verlauf der Dosierung eines Medikamentes ist in vielen Indikationsgebieten (z. B. Anästhesie, Diabetes, Schock, Sepsis, Herz-Kreislauf-Versagen, Asthma, Krebs) ausschlaggebend  für die Sicherheit und den Erfolg der Behandlung. Mithilfe von elektronisch gesteuerten Infusionspumpen können Arzneistoffe mit einer beliebig vorgegebenen zeitlich variablen Rate verabreicht werden. Das resultierende Konzentrations-Zeit-Profil und Effekt-Zeit-Profil hängt aber nicht nur von dem Dosierungsprofil ab, sondern es ist wesentlich durch die pharmakokinetischen (PK) und pharmakodynamischen (PD) Eigenschaften der jeweiligen Substanz bestimmt. Physiologiebasierte pharmakokinetische (PBPK) und pharmakodynamische Computermodelle sind prinzipiell in der Lage, das Konzentrations-Zeit-Profil sowie das Effekt-Zeit-Profil einer chemischen Substanz im Körper eines Patienten unter Berücksichtigung individueller physiologischer, anatomischer, biochemischer und genetischer Parameter zu simulieren.

Die Erfindung betrifft daher eine Vorrichtung in welchem ein PBPK/PD Modell verwendet wird, um einen für den individuellen Patienten optimalen zeitlichen Verlauf der Dosierung durch iterative Anpassung entweder des Konzentration-Zeit-Verlaufs im Plasma oder am Zielort (Target) oder des pharmakodynamischen Effekt-Zeit-Verlaufs an ein vorgegebenes zeitliches Zielprofil zu ermitteln. Dieser optimierte zeitliche Verlauf der Verabreichung dient dann als Eingangsfunktion für eine Dosierungsvorrichtung. In Kombination mit Echtzeit-Messungen physiologischer Parameter lässt sich ein geschlossener Regelkreis verwirklichen, der den im Stand der Technik genannten Blackbox-Verfahren, die auf die Nutzung physiologischen Wissens verzichten, deutlich überlegen ist.

Besonders vorteilhaft ist die weitere Kombination des Obigen mit einer Echtzeit-Messung von physiologischen Parametern, die zeitlichen Schwankungen unterliegen können, wie es z.B. im Falle der Expression von metabolisch aktiven Enzymen der Fall ist.

Das wesentliche Merkmal der Erfindung besteht in der Kombination eines physiologie-basierten Pharmakokinetik- und/oder Pharmakodynamik-Modells (PBPK/PD) mit einer automatisierten Dosiervorrichtung wie z. B. einer elektronisch gesteuerten Infusionspumpe. PBPK/PD-Modelle sind gegenüber nicht-physiologischen Kompartimentmodellen vorteilhaft, da sie in der Lage sind, den Einfluss individueller physiologischer, anatomischer, biochemischer und genetischer Faktoren auf die Pharmakokinetik und -dynamik detailliert zu beschreiben.

Durch Verwendung der PBK/PD Modelle ist es überraschenderweise nun möglich sehr individuelle, an den Patienten angepasste Dosierraten zu bestimmen.

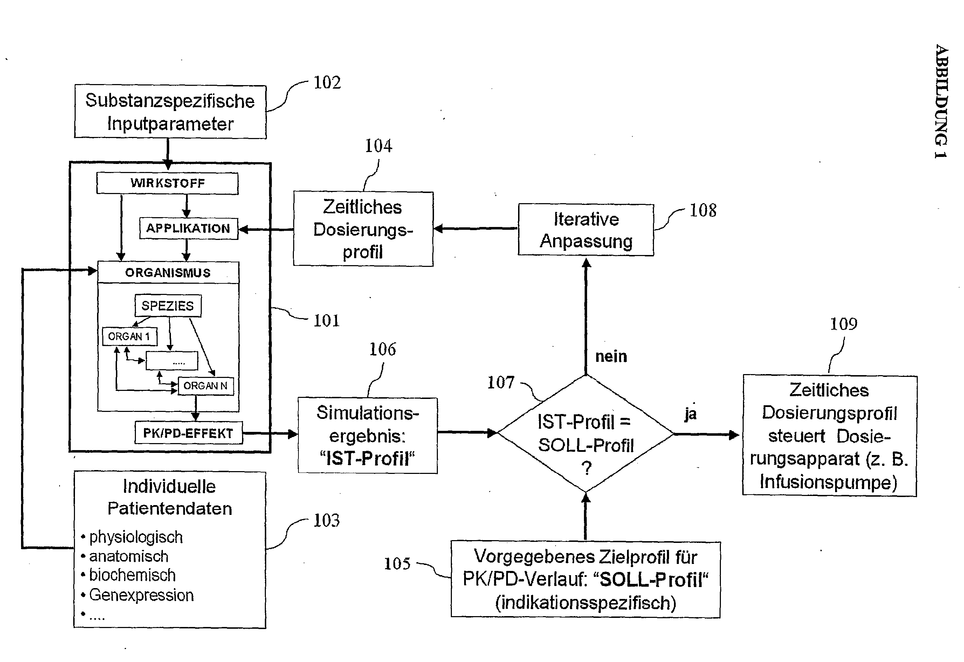

Eine schematische Darstellung eines Verfahrens auf der die erfindungsgemäße Vorrichtung basiert ist in Abbildung 1 gezeigt. Hauptbestandteil ist ein PBPK/PD-Modell (101), welches eine bestimmte Art von Organismus, besonders bevorzugt den eines Säugetieres, beschreibt und als Eingangsgrößen eine Reihe unterschiedlicher Parameter benötigt:

1.) Substanzspezifische Parameter des zu verabreichenden Arzneistoffes (102). Typische substanzspezifische Parameter sind z. B. physikochemische Parameter wie Lipophilie, Bindungskonstante an humanes Serumalbumin und/oder weitere Plasmaproteine, ungebundene Plasmafraktion, Löslichkeit in wässriger Pufferlösung oder in intestinaler Flüssigkeit, Größe des Moleküls (ausgedrückt durch das Molekülgewicht oder Molvolumen), hepatische und/oder renale Clearance, Permeabilitätskoeffizienten, bspw. über künstliche oder biologische Membranen, und Gleichgewichtsverteilungskoeffizienten zwischen Plasma (oder Blut) und den diversen Organen.

2.) Spezies-spezifische physiologische, anatomische, biochemische und/oder genetische Eingangsparameter, die charakteristisch für den betrachteten Patienten sind (103). Zu dieser Sorte von Parametern zählen insbesondere Körpergewicht, Volumenanteile einzelner Organe am gesamten Körpervolumen, Blutflussraten einzelner Organe, Wasser-, Fett- und Lipidanteil der einzelnen Organe, sowie Parameter, die die Expression und Funktion von metabolisch aktiven Enzymen (insbesondere in Leber und Darm) oder die Expression und Funktion von Proteinen für den aktiven Transport von Molekülen durch Zellmembranen charakterisieren. Diese Parameter können entweder direkt gemessen werden oder sind für bestimmte Populationen mit einfach bestimmbaren Patientenparametern wie Alter, Geschlecht, Körpergewicht und Lean Body Mass korreliert (P. S. Price, R. B. Conolly, C. F. Chaisson, E. A. Gross, J. S. Young, E. T. Mathis, D. R. Tedder: "Modeling interindividual variation in physiological factors used in PBPK models of humans", Crit. Rev. Toxicol. 33, 469-503 (2003)). Es können einzelne oder mehrere dieser Parameter im erfindungsgemäßen Verfahren eingesetzt werden.

3.) Ein Dosierungsprofil, dass die verabreichte Dosis als Funktion der Zeit beschreibt (104).

Weiter wird vorgegeben:

4.) Ein Zielprofil, das den gewünschten Konzentrations-Zeit-Verlauf des zu verabreichenden Arzneistoffs im Plasma, Blut, oder direkt am biochemischen Target im Zielorgan oder den gewünschten Effekt-Zeit-Verlauf angibt ("SOLL-Profil", 105). Dieses Zielprofil ist indikationsspezifisch und abhängig von dem betrachteten Arzneistoff. In vielen klinisch relevanten Fällen wie beispielsweise bei der Verabreichung von Narkosemitteln wird ein rechteckiges Effekt-Profil mit möglichst steilen Flanken angestrebt, d. h. die gewünschte Wirkung (hier: Narkose) soll spontan einsetzen, dann über einen definierten Zeitraum möglichst konstant bleiben, und am Ende der Behandlung wieder rasch abklingen. Das Zielprofil kann entweder eine einfache zeitliche Funktion $Z(t)$ sein oder auch alternativ oder zusätzlich als Toleranzbereich (definiert als Intervall über einen Maximalwert und einen Minimalwert $[Z_{min}(t)..Z_{max}(t)]$) vorgegeben sein.

Ausgehend von einer sinnvoll zu wählenden Startfunktion für das Dosierungsprofil, errechnet das PBPK/PD-Modell anhand der Informationen aus 1.) - 2.) das individuelle Konzentrations-Zeit-Profil bzw. Effekt-Zeit-Profil für die betrachtete Substanz ("IST-Profil", 106).

Dabei sind grundsätzlich verschiedene Routen der Administration des Arzneistoffs im PBPK/PD-Modell denkbar. Der Arzneistoff kann intravenös, intraarteriell, intraperitoneal, intramuskulär, subkutan, topikal, oral oder inhalativ über die oberen Atemwege und/oder die Lunge verabreicht werden. Besonders wichtig und bevorzugt ist die intravenöse Applikation.

Der nächste Schritt des Verfahrens besteht aus einem iterativen Optimierungsprozess, in dem das Dosierungsprofil solange variiert wird, bis das simulierte Konzentrations-Zeit-Profil bzw. Effekt-Zeit-Profil mit dem SOLL-Profil übereinstimmt (107, 108). Als Resultat dieser numerischen Optimierung wird dasjenige zeitliche Dosierungsprofil erhalten, welches den gewünschten Konzentrations-Zeit- bzw. Effekt-Zeit-Verlauf für die betrachtete Substanz bei dem individuellen Patienten bewirkt oder von diesem die geringste Abweichung zeigt. Als numerische Optimierungsverfahren kommen zum Beispiel Gradientenverfahren, gradientenfreie Verfahren, stochastische Verfahren oder evolutionäre Verfahren in Betracht. Besonders bevorzugt sind bei den Gradientenverfahren das Quasi-Newton- oder Newton-Verfahren und insbesondere das Intervallschachtelungsverfahren bei den gradientenfreien Verfahren. Bei den stochastischen Methoden ist insbesondere das Monte-Carlo-Verfahren bevorzugt, die Methode der genetischen Optimierung stellt eine besonders bevorzugte Form eines evolutionären Verfahrens dar.

Dieses Dosierungsprofil wird im letzten Schritt zur Steuerung einer automatischen Dosierungsvorrichtung herangezogen.

Dementsprechend betrifft die vorliegende Erfindung also eine Vorrichtung zur zeitlich gesteuerten Dosierung eines Arzneistoffs in der folgende Schritte angewendet werden:

a) Vorgabe eines indikations- und substanzabhängigen Zielprofils, welches einen gewünschten Konzentrations-Zeit-Verlauf oder einen gewünschten Effekt-Zeitverlauf angibt,

b) Physiologie-basierte pharmakokinetische und/oder pharmakodynamische Simulation mit zeitlich variablem Applikationsprofil unter Berücksichtigung individueller Parameter des Patienten und substanzspezifischer Inputparameter des zu verabreichenden Arzneistoffes,

c) Iterative numerische Anpassung des Applikationsprofils, bis das simulierte zeitliche Profil mit dem vorgegebenen Zielprofil übereinstimmt oder mit diesem die maximal erreichbare Übereinstimmung zeigt,

d) Steuerung einer Dosierungsvorrichtung auf Basis des Ergebnisses unter c).

Besonders vorteilhaft ist das erfindungsgemäße Vorrichtung, wenn physiologische Parameter, die einen Einfluss auf das pharmakokinetische oder pharmakodynamische Verhalten des Arzneistoffes haben, zeitlich variabel sind. Zu den physiologischen Parametern, die sich während des Therapieverfahrens ändern, zählen beispielsweise die Blutflüsse des Magen-Darm-Trakts und der Leber, die unter anderem durch die Aufnahme von Nahrung beeinflusst und verändert werden, oder (auf einer längeren Zeitskala) auch die Exprimierungsraten von Transportern oder metabolisch aktiven Enzymen. Sofern solche Parameter in Echtzeit während der Therapie gemessen werden, kann deren zeitlich veränderlicher Einfluss bei der Optimierung des Dosierungsprofils mit der erfindungsgemäßen Vorrichtung mitberücksichtigt werden.

In bestimmten Fällen wie z. B. in der Anästhesie kann es sinnvoll sein, die Regelung der Dosierungsvorrichtung nicht alleine auf das gewünschte pharmakokinetische oder pharmakodynamische Zielprofil hin vorzunehmen, sondern eine externe Messgröße als weiteren Eingangsparameter heranzuziehen.

In einer besonderen Ausführungsform wird der Therapieerfolg zusätzlich durch eine oder mehrere geeignete Messsonden, die z. B. die Narkosetiefe bei einer Anästhesie messen, online überwacht und die Messsignale als zusätzliche Eingangsgrößen in das Verfahren integriert. Die Steuerung der Dosierungsvorrichtung wird dann nicht rein durch das pharmakodynamische oder pharmakokinetische Zeitprofil geregelt, sondern bezieht externe Messsignale mit ein. So kann beispielsweise die Zufuhr eines Narkosemittels erhöht werden, wenn die gemessene Narkosetiefe einen kritischen Wert unterschreitet. Auf diese Weise lässt sich ein geschlossener Regelkreis verwirklichen, der unter Einbeziehung von online Messwerten und physiologischen Simulationen in Echtzeit die zeitliche Dosierung des Arzneimittels optimiert. In dieser Ausführungsform wird die aus der physiologischen Simulation bekannte Response des pharmakodynamischen bzw. pharmakokinetischen Profils auf Änderungen in der Applikationsrate benutzt, um die Rate der Arzneistoffzufuhr den durch die Messsonde angezeigten Bedürfnissen des Patienten neu anzupassen. In einer weiteren besonderen Ausführungsform wird kurzzeitig alleine das Signal der Messsonde zur Regelung herangezogen, sofern ein kritischer Zustand vorliegt (z. B. wenn der Patient vorzeitig aus der Narkose zu erwachen droht).

Als Simulationsverfahren sind grundsätzlich alle auf den genannten Parametern basierenden Verfahren geeignet, besonders geeignet und erfindungsgemäß bevorzugt sind die in DE Anm. Nr. 10160270 und DE Anm. Nr. 10345836 beanspruchten Verfahren.

Neben der Anwendung der erfindungsgemäßen Vorrichtung als Hilfsmittel zur Durchführung einer medizinischen Therapie lässt sich das erfindungsgemäße Verfahren auch bereits in klinischen Versuchen oder Tierversuchen als Hilfsmittel einsetzen, bspw. um in die Testreihen mit klinisch "sinnvollen" Dosierungen einzusteigen und das typische "Einpendeln" der Dosierung, also die empirisch-iterative Annäherung von zu großen über zu kleine Dosierungen, die sich abwechselnd an das Optimum annähern, auf ein Minimum zu reduzieren und damit die Belastung der behandelten Organismen zu minimieren und die Wahrscheinlichkeit eines Erfolgs des Experiments zu maximieren.

Als Zielgruppe für die Anwendung der erfindungsgemäßen Vorrichtung kommen dementsprechend Menschen und Tiere, insbesondere Menschen sowie Nutz-, Zucht-, Labor-, Versuchs- und Hobbytiere in Frage. Ganz besonders bevorzugt wird die Vorrichtung als Hilfsmittel für die therapeutische Behandlung von Menschen oder klinischen Versuchen an Menschen eingesetzt.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Tauben, Vogelarten für Heim- und Zoohaltung.

Zu Labor- und Versuchstieren gehören Mäuse, Ratten, Meerschweinchen, Hamster, Kaninchen, Hunde, Katzen, Schweine und Affen jeweils in allen Arten, Unterarten und Rassen.

Zu den Hobbytieren gehören Hunde und Katzen.

Als elektronisch gesteuerte Dosierungsvorrichtung kommen elektronisch gesteuerte Infusionspumpen, Inhalatoren oder elektronisch gesteuerte Freisetzungskapseln für die orale Applikation in Frage. Besonders geeignet sind elektronisch gesteuerte Infusionspumpen.

Die erfindungsgemäße Vorrichtung ist besonders vorteilhaft bei solchen Indikationen und Wirkstoffen, die nur über ein schmales "therapeutisches Fenster" verfügen. Ein schmales therapeutisches Fenster bedeutet, dass nur ein kleiner Konzentrationsbereich existiert, bei dem zwar die erwünschten pharmakologischen Effekte auftreten, aber gleichzeitig noch keine unerwünschten Nebenwirkungen zu beobachten sind. Beispiele für Indikationsgebiete mit schmalem therapeutischen Fenster sind alle Arten von Krebserkrankungen, Infektionskrankheiten, insbesondere bakterielle und virale Infektionen, Herz-Kreislauferkrankungen, insbesondere Bluthochdruck. Lipidämie, Angina Pectoris und Herzinfarkt, Erkrankungen des Zentralnervensystems wie Morbus Alzheimer, Schizophrenie, Epilepsie, chronische Kopfschmerzen (Migräne), Schmerztherapie und Anästhesie, psychische Erkrankungen, insbesondere Depressionen und Angstzustände, Stoffwechselerkrankungen wie z. B. Diabetes und Störungen des Fettstoffwechsels (Adipositas), Atemwegserkrankungen wie Asthma und Bronchitis, Immunerkrankungen, insbesondere Allergien, Rheuma und Multiple Sklerose, Erkrankungen des Magen-Darm Traktes, insbesondere Geschwüre des Magens und des Zwölffingerdarms und Morbus Crohn, sowie Erkrankungen der Gefäße, insbesondere solche, die zur erektalen Dysfunktion führen, und akute Schockzustände.

**Beispiel**

Die Erfindung wird im Folgenden anhand eines Beispiels näher erläutert, dieses Beispiel illustriert die vorliegende Erfindung ist jedoch keinesfalls als Einschränkung zu betrachten.

Dem Beispiel liegen Simulationen mit dem bei Bayer Technology Services entwickelten physiologie-basierten Pharmakokinetik-Modell PK-Sim® zu Grunde.

Als Beispielsubstanz wurde Theophyllin, ein Arzneistoff zur Therapie von Asthma, gewählt. Das besondere an dieser Substanz ist, dass sie nur über einen sehr schmalen Konzentrationsbereich verfügt, in dem die Substanz therapeutisch wirksam ist. Dieses sog. "therapeutische Fenster" wird in der Literatur mit 5-10 bis 20 mg/L (Plasma-Konzentration) angegeben (A. Ohnishi: "A Review of Clinical Use of Theophylline in Acute Asthma: Factors Influencing Kinetic Disposition and Drug Interactions", Methods Find. Exp. Clin. Pharmacol. 22, 253-258 (2000)). Bei Plasma-Konzentrationen unterhalb von 5 mg/L wird im allgemeinen keine ausreichende anti-asthmatische Wirkung erzielt, oberhalb einer Plasma-Konzentration von 20 mg/L steigt das Risiko unerwünschter Nebenwirkungen wie Übelkeit, Zittern und beschleunigter Herzschlag stark an (A. Ohnishi: "A Review of Clinical Use of Theophylline in Acute Asthma: Factors Influencing Kinetic Disposition and Drug Interactions", Methods Find. Exp. Clin. Pharmacol. 22, 253-258 (2000)). Erschwerend kommt beim therapeutischen Einsatz von Theophyllin der bekannte Umstand hinzu, dass die Pharmakokinetik dieses Arzneistoffs stark von individuellen Faktoren des Patienten wie z. B. Alter, Geschlecht, oder Körperfettanteil abhängt. Besonders die Metabolisierungsrate (P. A. Mitenko, R. I. Ogilvie: "Pharmacokinetics of intravenous Theophylline", Clin. Pharm. Therap. 14, 509-513 (1973); J. P. Jameson, A. Munyika: "Theophylline Pharmacokinetics in Black Zimbabwean Males", Therap. Drug Monitoring 12, 54-58 (1990); P. Gal, W. J. Jusko, A. M. Yurchak, B. A. Franklin: " Theophylline disposition in obesity", Clin. Pharmacol. Ther. 23, 438-44 (1978); S. H. D. Jackson, A. Johnston, R. Woollard, P. Turner: "The Relationship between Theophylline Clearance and Age in Adult Life", Eur. J. Clin. Pharmacol. 36, 29-34 (1989)), sowie die freie Plasmafraktion (Z. Trnavska: "Theophylline Protein Binding", Arzneim.-Forsch. 40, 166-170 (1990); Z. Kato, O. Fukutomi, N. Kondo: "Developmental Changes of unbound Theophylline", Ann Allergy Asthma Immunol. 80, 517 (1998)), sind individuell unterschiedlich und führen folglich zu individuell unterschiedlichen Plasma-Konzentrationsverläufen. Im klinischen Einsatz wird man diesem Problem dadurch gerecht, dass die Plasma-Konzentration von Theophyllin experimentell überwacht wird (A. Ohnishi: "A Review of Clinical Use of Theophylline in Acute Asthma: Factors Influencing Kinetic Disposition and Drug Interactions", Methods Find. Exp. Clin. Pharmacol. 22, 253-258 (2000)). Dieses Verfahren ist vergleichsweise aufwändig, es erfordert wiederholte Entnahmen von Blutproben beim Patienten und ist darüber hinaus nur bei der Langzeittherapie mit Theophyllin sinnvoll anzuwenden.

Wie das Problem einer individuell auf den Patienten zugeschnittenen, optimalen Dosierung von Theophyllin mit dem erfindungsgemäßen Verfahren gelöst werden kann, wird im folgenden beschrieben:

Als substanzabhängige Inputparameter werden die folgenden Werte für Theophyllin verwendet (Tabelle 1):

Tabelle 1

| PARAMETER | WERT |
|---|---|
| Lipophilie (LogMA) | -0.2 |

(fortgesetzt)

| PARAMETER | WERT |
|---|---|
| Freie Plasmafraktion | 59 % |
| Löslichkeit | 2800 mg/L |
| Molgewicht | 180 Dalton |
| Intestinale Permeabilität (nur bei oraler Aufnahme, Abb. 2c) | $2.97 \times 10^{-6}$ cm/s |

Der verwendete Wert von 59 % für die freie Plasmafraktion stellt einen mittleren Wert für einen erwachsenen Menschen dar (Z. Trnavska: "Theophylline Protein Binding", Arzneim.-Forsch. 40, 166-170 (1990); Z. Kato, O. Fukutomi, N. Kondo: "Developmental Changes of unbound Theophylline", Ann Allergy Asthma Immunol. 80, 517 (1998)). Weiterhin wurden die folgenden physiologischen und anatomischen Parameter bei den Berechnungen mit PK-Sim® zugrunde gelegt (Default-Parameter für den Menschen, Tabelle 2):

Tabelle 2

| Organ / Kompartiment | Volumina [ml] | Blutflussraten [ml/min] |
|---|---|---|
| venöser Blutpool | 250 | -/- |
| arterieller Blutpool | 140 | -/- |
| Lunge | 670 | 4670 |
| Magen | 150 | 60 |
| Dünndarm | 640 | 600 |
| Dickdarm | 370 | 240 |
| Pankreas | 100 | 60 |
| Milz | 180 | 180 |
| Leber | 1710 | 390 |
| Gallenblase | 20 | 0 |
| Niere | 720 | 1133 |
| Gehirn | 1486 | 700 |
| Herz | 330 | 240 |
| Muskel | 30200 | 550 |
| Knochen | 12060 | 167 |
| Haut | 3020 | 50 |
| Fett | 10060 | 300 |
| Hoden | 35 | 2.6 |

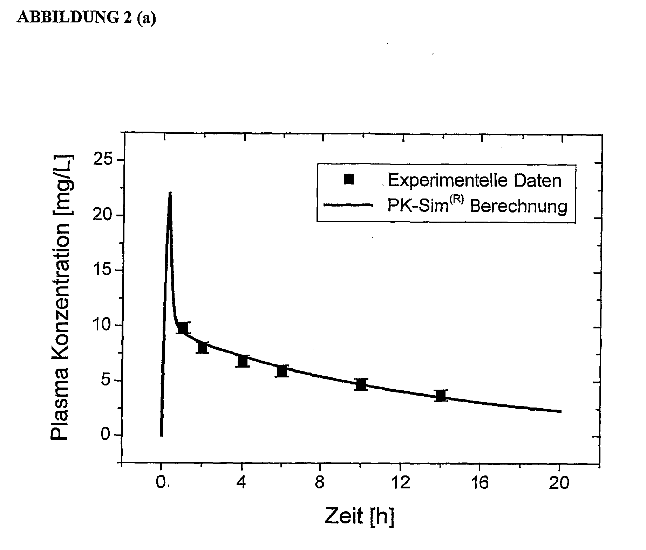

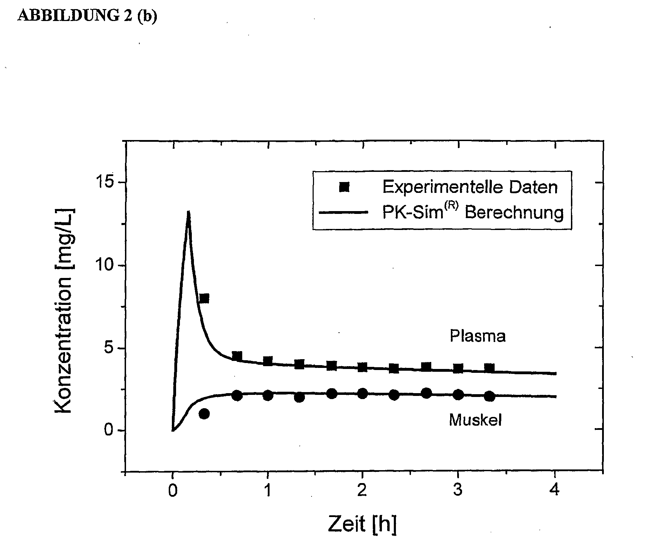

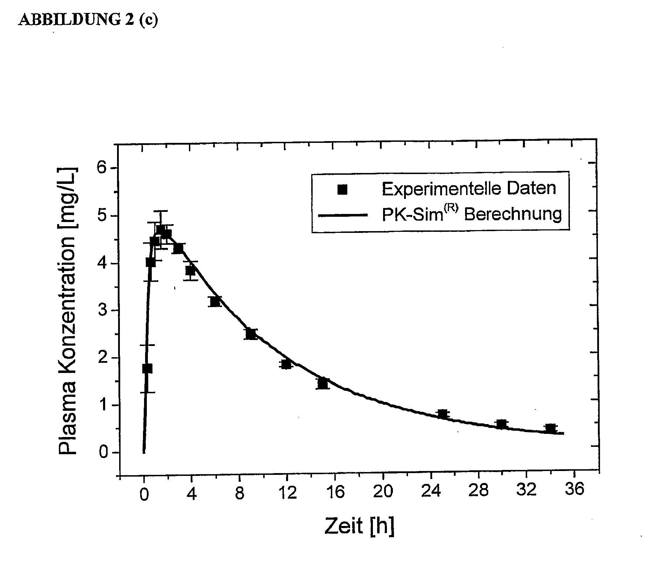

Zunächst wird gezeigt, dass es grundsätzlich auf der Basis der vorgenannten Werte möglich ist, mit PK-Sim® den Plasma-Konzentrations-Zeitverlauf von Theophyllin korrekt zu beschreiben. Dazu wurden Simulationsergebnisse mit experimentell gemessenen Plasma-Konzentrations-Zeitverläufen (S. Valenti, P. Crimi, V. Brusasco: "Bioavailability and Pharmacokinetics of a New Controlled Release Theophylline Preparation in the Form of Capsules Containing Pellets", Respiration 52, 195-200 (1987); M.Müller, B. v. Osten, R. Schmid, E. Piegler, I. Gerngross, H. Buchegger, H. G. Eichler: "Theophylline kinetics in peripheral tissues in vivo in humans", Naunyn-Schmiedeberg's Arch. Pharmacol. 352, 438-441 (1995); M. C. Meyer, E. J. Jarvi, A. B. Straughn, F. R: Pelsor, R. L. Williams, V. P. Shah: "Bioequivalence of Immediate-release Theophylline Capsules", Biopharm. Drug Dispos. 20, 417-419 (1999)) und einem Muskel-Konzentrations-Zeitverlauf aus der Literatur (M.Müller, B. v. Osten, R. Schmid, E. Piegler, I. Gerngross, H. Buchegger, H. G. Eichler: "Theophylline kinetics in peripheral tissues in vivo in humans", Naunyn-Schmiedeberg's Arch. Pharmacol. 352, 438-441 (1995)) verglichen (Abbildung 2). Wie in Abbildung 2 zu sehen ist, ist die Übereinstimmung der simulierten Kurven mit den experimentell bestimmten Kurven sehr gut.

Zur Optimierung von individuellen Dosierungsprofilen werden im folgenden zwei virtuelle Individuen betrachtet, die sich beispielhaft in ihrer gesamten Metabolisierungsrate ("Clearance") unterscheiden. Individuum A weist eine totale Plasma Clearance (CL) von 0.4 ml/min/kg, Individuum B von 1.0 ml/min/kg auf. Diese Werte liegen innerhalb der typischen Schwankungsbreite derPlasma-Clearance von Theophyllin im Menschen (P. A. Mitenko, R I. Ogilvie: "Pharmacokinetics_ of intravenous Theophylline", Clin. Pharm. Therap. 14, 509-513 (1973); J. P. Jameson, A. Munyika: "Theophylline Pharmacokinetics in Black Zimbabwean Males", Therap. Drug Monitoring 12, 54-58 (1990); P. Gal, W. J. Jusko, A. M. Yurchak, B. A. Franklin: " Theophylline disposition in obesity", Clin. Pharmacol. Ther. 23, 438-44 (1978); S. H. D. Jackson, A. Johnston, R. Woollard, P. Turner: "The Relationship between Theophylline Clearance and Age in Adult Life", Eur. J. Clin. Pharmacol. 36, 29-34 (1989)). Die übrigen relevanten, physiologischen und anatomischen Parameter, die in den folgenden Simulationen verwendet werden, sind für beide Individuen gleich angenommen und identisch mit den in Tabelle 2 zusammengefassten Werten. Denkbar ist an dieser Stelle natürlich auch die Berücksichtigung von einer Vielzahl weiterer individueller Unterschiede wie z. B. in der freien Plasmafraktion, im Körpergewicht, im Fettanteil des Körpers, oder in der Blutflussrate in einem oder mehreren Organen.

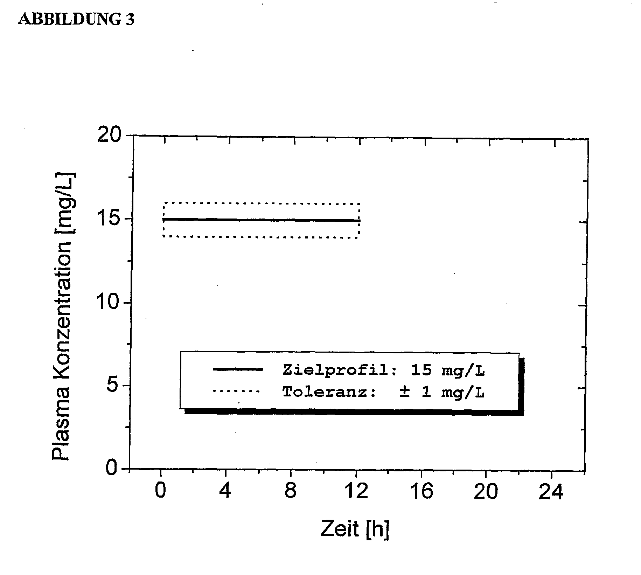

Als SOLL-Profil wird in diesem Beispiel eine Plasma-Konzentration von 15 mg/L gewählt, die über einen Zeitraum von 12 Stunden konstant gehalten werden soll. Der gewählte Toleranzbereich beträgt $\pm$ 1 mg/L (dargestellt in Abbildung 3), d. h. es gilt

$$Z(t) = \begin{cases} 0 & , \ t < 0 \\ 15\,\mathrm{mg/L} & , \ 0 \leq t \leq 12\,\mathrm{h} \qquad \text{sowie} \\ 0 & , \ t > 12\,\mathrm{h} \end{cases} \tag{1}$$

$$Z_{\min}(t) = \begin{cases} 0 & , \ t < 0 \\ 14\,\mathrm{mg/L} & , \ 0 \leq t \leq 12\,\mathrm{h} \\ 0 & , \ t > 12\,\mathrm{h} \end{cases} \quad \text{und} \quad Z_{\max}(t) = \begin{cases} 0 & , \ t < 0 \\ 16\,\mathrm{mg/L} & , \ 0 \leq t \leq 12\,\mathrm{h} \\ 0 & , \ t > 12\,\mathrm{h} \end{cases} .$$

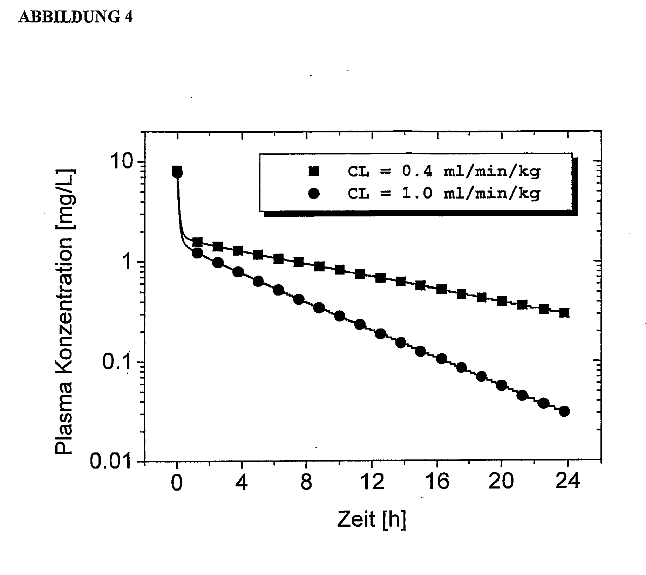

In Abbildung 4 sind die mit PK-Sim® simulierten Plasma-Konzentrations-Zeitverläufe für die beiden Individuen nach einer intravenösen Bolus-Administration von 1 mg/kg Theophyllin gezeigt. Diese Kurven dienen als Startpunkt für die Optimierung des Dosierungsprofils.

Im nächsten Schritt wird nun iterativ die Infusionsrate, d. h. die intravenös applizierte Dosis pro Zeitintervall, variiert. Die Schrittweite des Dosierungsintervalls beträgt in diesem Beispiel 0.1 Stunde = 6 Minuten. Diese Größe ist sinnvollerweise so zu wählen, dass sie an die Verteilungs- und Eliminationskinetik der Substanz angepasst wird.

Das erhaltene Simulationsergebnis (= IST-Profil) wird dann mit dem SOLL-Profil verglichen. Im Falle einer Abweichung des IST-Profils vom SOLL-Profil, die außerhalb des gültigen Toleranzbereichs liegt, wird die im entsprechenden Zeitschritt applizierte Menge variiert, bis insgesamt das IST-Profil im Rahmen des Toleranzbereichs mit dem SOLL-Profil übereinstimmt. Im vorliegenden Beispiel wurde die Optimierung des Applikationsprofils durch manuelle Iteration vorgenommen. Im Rahmen einer echten Therapieoptimierung sind an dieser Stelle geeignete numerische Optimierungsverfahren einzusetzen. Als geeignete Optimierungsverfahren kommen hier beispielsweise Gradientenverfahren, insbesondere Quasi-Newton- oder Newton-Verfahren, sowie gradientenfreie Verfahren wie Intervallschachtelung und stochastische Verfahren wie Monte-Carlo-Verfahren in Betracht.

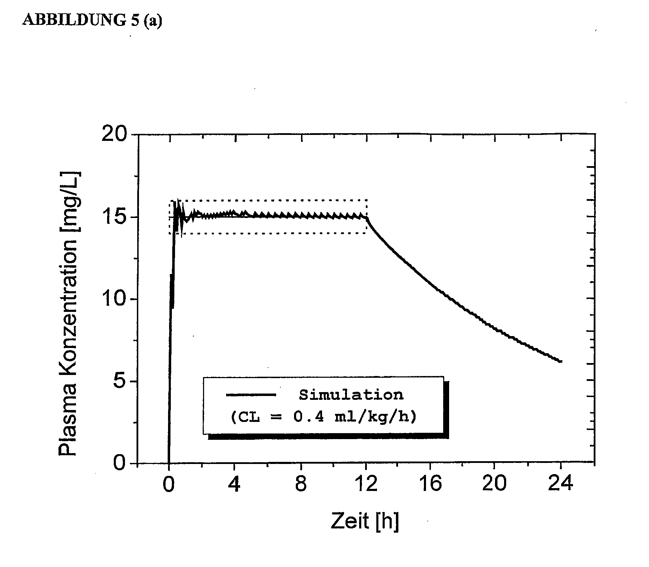

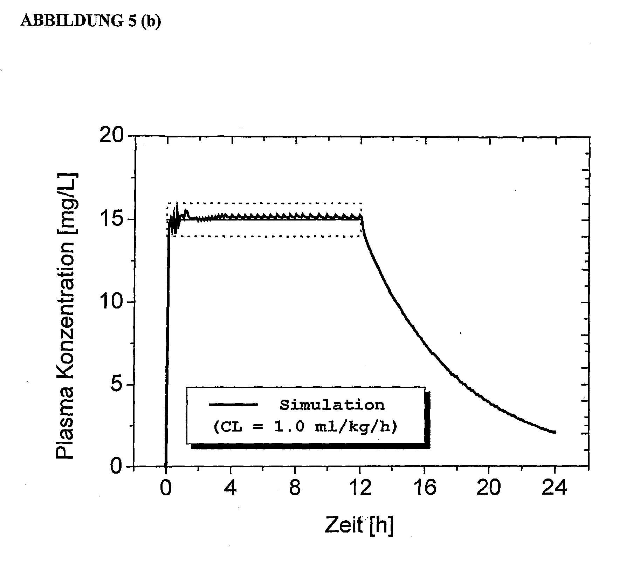

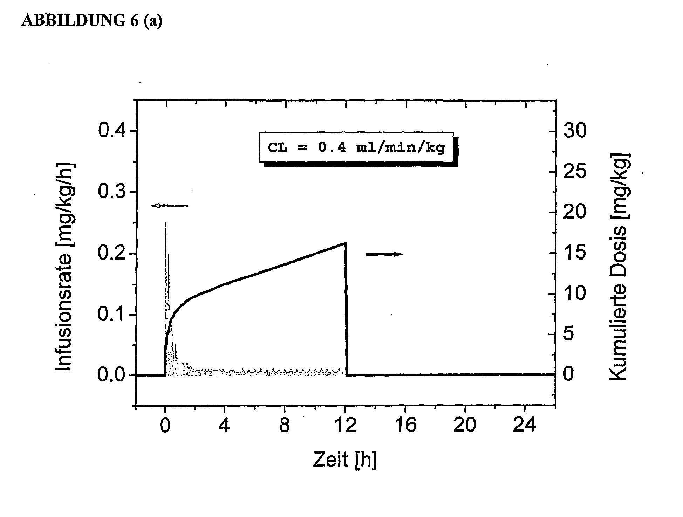

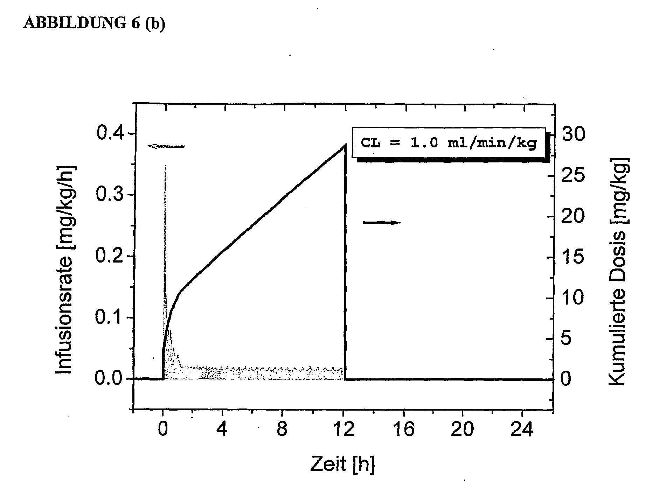

Ein jeweiliges Ergebnis eines solchen manuellen iterativen Optimierungsprozesses ist in den Abbildungen 5 (a) und (b) dargestellt. Die zu diesen IST-Profilen gehörenden Dosierungsprofile sind in den Abbildungen 6 (a) und (b) gezeigt. Diese so ermittelten Dosierungsprofile dienen im letzten Schritt des Verfahrens als Eingabefunktion zur Steuerung eines herkömmlichen Dosierungsautomaten, z. B. einer elektronisch gesteuerten Infusionspumpe.

**Beschreibung der Abbildungen**

**Abbildung 1:** Schematische Darstellung des Verfahrens zur zeitlichen Dosierung von Arzneistoffen.

**Abbildung 2:** Vergleich zwischen experimentellen Konzentrations-Zeitverläufen von Theophyllin aus der Literatur mit Simulationsergebnissen von PK-Sim® mit an die jeweiligen experimentellen Daten angepassten Metabolisierungsraten:

a) Intravenöse Infusion von 5.58 mg/kg über 20 Minuten. Die Plasma-Clearance beträgt hier CL = 0.40 ml/min/kg. (Quelle: [12])

b) Intravenöse Infusion von 240 mg über 10 Minuten. Die Plasma-Clearance beträgt hier CL = 0.25 ml/min/kg. (Quelle: [13])

c) Perorale Administration von 200 mg einer sofort freisetzenden Formulierung (Mittelwert und Standardabweichung dreier bioäquivalenter *immediate release*-Kapseln [14]). Die
Plasma-Clearance beträgt hier CL = 0.50 ml/min/kg.

**Abbildung 3:** Vorgabe des Zielprofils (= SOLL-Profil)

**Abbildung 4:** Simulationsergebnis für die intravenöse Bolus-Administration von 1 mg/kg Theophyllin im Individuum A (CL = 0.4 ml/min/kg) und B (CL = 1.0 ml/min/kg).

**Abbildung 5:** Simuliertes Plasma-Konzentrations-Zeitprofil (IST-Profil) nach iterativer Anpassung des Dosierungsprofils für Individuum A **(a)** und B **(b).**

**Abbildung 6:** Optimiertes Dosierungsprofil für Individuum A **(a)** und B **(b).**

**Patentansprüche**

1. Vorrichtung zur zeitlichen Steuerung der Dosierung eines Arzneistoffs, umfassend die Kombination eines physiologie-basierten Pharmakokinetik- und Pharmakodynamik-Modells mit einer automatisierten Dosiervorrichtung, das zur Durchführung der folgenden Schritte hergerichtet ist:

   a) Physiologie-basierte pharmakokinetische und pharmakodynamische Simulation mit zeitlich variablem Applikationsprofil unter Berücksichtigung individueller anatomischer, physiologischer und/oder genetischer Parameter des zu behandelnden Organismus ausgewählt aus der Gruppe bestehend aus Blutflussraten, Volumina und Zusammensetzung einzelner Organe, Genexpressionsdaten von metabolisch aktiven Enzymen oder aktiven Transportern und substanzspezifischer Inputparameter des zu verabreichenden Arzneistoffes, auf Basis eines vorzugebenden indikations- und substanzabhängigen Zielprofils, welches einen gewünschten Konzentrations-Zeit-Verlauf oder einen gewünschten Effekt-Zeit-Verlauf angibt,
   b) Iterative numerische Anpassung des Applikationsprofils, bis das simulierte zeitliche Profil mit dem vorgegebenen Zielprofil übereinstimmt,
   c) Steuerung der Dosierungsvorrichtung auf Basis des Ergebnisses unter b).

2. Vorrichtung nach Anspruch 1, wobei es sich bei der Art der Applikation um eine der folgenden Arten handelt: Intravenöse Applikation, intraarterielle Applikation, intraperitoneale Applikation, intramuskuläre Applikation, subkutane Applikation, topikale Applikation, orale Applikation oder inhalative Applikation.

3. Vorrichtung nach einem der Ansprüche 1 bis 2, wobei es sich bei den zu berücksichtigenden substanzspezifischen Parametern um eine Auswahl aus den folgenden handelt: Lipophilie, Bindungskonstante an Plasmaproteine, freie Fraktion im Plasma, Löslichkeit, Permeabilitätskoeffizient, Molgewicht, Molvolumen, Organ/Plasma- bzw. Organ/Blut-Verteilungskoeffizient.

4. Vorrichtung nach einem der Ansprüche 1 bis 3, wobei als Methode zur Anpassung des Applikationsprofils eines der folgenden numerischen Optimierungsverfahren zum Einsatz kommt: Gradientenverfahren, insbesondere Quasi-Newton- oder Newton-Verfahren; Gradientenfreie Verfahren wie Intervallschachtelung; stochastische Verfahren wie Monte-Carlo-Verfahren.

5. Vorrichtung nach einem der Ansprüche 1 bis 4, wobei es sich bei der Dosierungsvorrichtung um eine elektronisch gesteuerte Infusionspumpe, einen Inhalator oder eine elektronisch gesteuerte Freisetzungskapsel für die orale Applikation handelt.

6. Vorrichtung nach Anspruch 1, ausgebildet zur Messung einer oder mehrere der anatomischen, physiologischen und/oder genetischen Parameter in Echtzeit während der Applikation.

7. Vorrichtung nach einem der Ansprüche 1 bis 6, ausgebildet um zusätzlich den Therapieerfolg online durch eine oder mehrere geeignete Messsonden zu überwachen und deren Messsignal bzw. Messsignale mit zur Steuerung der Dosierungsvorrichtung heranzuziehen.

**EP 1 722 839 B1**

**Claims**

1. Device for controlling the dosage of a medicament as a function of time, comprising the combination of a physiology-based pharmacokinetic and pharmacodynamic model with an automated dosage device, said model being readied for carrying out the following steps:

   a) Physiology-based pharmacokinetic and pharmacodynamic simulation with a time-variable application profile while taking into account individual anatomical, physiological and/or genetic parameters of the body to be treated, selected from the group consisting of blood flow rates, volumes and composition of individual organs, gene expression data of metabolically active enzymes or active transporters, and substance-specific input parameters of the medicament to be administered, based on an indication- and substance-dependent target profile to be specified, which indicates a desired concentration-time profile or a desired effect-time profile,
   b) Iterative numerical adaptation of the application profile until the simulated time profile matches the predetermined target profile,
   c) Control of the dosage device on the basis of the result in b).

2. Device according to Claim 1, wherein the type of application is one of the following types: intravenous application, intra-arterial application, intraperitoneal application, intramuscular application, subcutaneous application, topical application, oral application or inhalative application.

3. Device according to any of Claims 1 to 2, wherein the substance-specific parameters to be taken into account are a selection from the following: lipophilicity, binding constants to plasma proteins, free fraction in plasma, solubility, permeability coefficient, molar mass, molar volume, organ/plasma or organ/blood distribution coefficient.

4. Device according to any of Claims 1 to 3, wherein one of the following numerical optimization methods is used as the method for adapting the application profile: gradient methods, in particular quasi-Newton or Newton methods; gradient-free methods such as nested intervals; stochastic methods such as Monte-Carlo methods.

5. Device according to any of Claims 1 to 4, wherein the dosage device is an electronically controlled infusion pump, an inhaler or an electronically controlled release capsule for oral application.

6. Device according to Claim 1, configured for measuring one or more of the anatomical, physiological and/or genetic parameters in real-time during the application.

7. Device according to one of Claims 1 to 6, configured for additionally monitoring the success of the therapy online by one or more suitable measurement probes and co-employing their measurement signal or measurement signals in order to control the dosage device.

**Revendications**

1. Dispositif permettant de commander en fonction du temps le dosage d'un médicament et comprenant la combinaison d'un modèle pharmacocinétique et d'un modèle pharmacodynamique à base physiologique et d'un dispositif automatique de dosage conçu pour exécuter les étapes suivantes :

   a) simulation pharmacocinétique et pharmacodynamique à base physiologique selon un profil d'application variable dans le temps en tenant compte de paramètres anatomiques, physiologiques et/ou génétiques de l'organisme particulier à traiter, qui sont sélectionnés dans l'ensemble constitué du débit sanguin, du volume et de la composition de certains organes, de données d'expression génétique d'enzymes métaboliquement actifs ou de transporteurs actifs et de paramètres d'entrée, spécifiques à la substance, du médicament à administrer, sur la base d'un profil-cible à déterminer en fonction de l'indication et de la substance et qui donne une évolution souhaitée de la concentration en fonction du temps ou une évolution souhaitée de l'effet en fonction du temps,
   b) adaptation numérique itérative du profil d'application jusqu'à ce que le profil temporel simulé corresponde au profil-cible prédéterminé, et
   c) commande du dispositif de dosage sur base du résultat de l'étape b).

2. Dispositif selon la revendication 1, dans lequel le type d'application est un des types suivantes : application intra-

9

veineuse, application intra-artérielle, application intra-péritonéale, application intramusculaire, application sous-cutanée, application topique, application orale ou application par inhalation.

3. Dispositif selon l'une des revendications 1 et 2, dans lequel les paramètres spécifiques à la substance qui doivent être pris en compte sont une sélection des paramètres suivants : lipophilie, constante de liaison à des protéines du plasma, fraction libre dans le plasma, solubilité, coefficient de perméabilité, poids moléculaire, volume moléculaire, coefficient de répartition dans les organes et le plasma ou dans les organes et le sang.

4. Dispositif selon l'une des revendications 1 à 3, dans lequel la méthode d'adaptation du profil d'application utilise l'un des procédés d'optimisation numérique suivant : procédé à gradient, en particulier procédé de quasi-Newton ou de Newton, procédé sans gradient, par exemple emboîtement d'intervalles et procédé stochastique tel que le procédé de Monte-Carlo.

5. Dispositif selon l'une des revendications 1 à 4, dans lequel le dispositif de dosage est une pompe de perfusion à commande électronique, un inhalateur ou une capsule de libération à commande électronique en vue d'une application orale.

6. Dispositif selon la revendication 1, configuré pour mesurer un ou plusieurs des paramètres anatomiques, physiologiques et/ou génétiques en temps réel pendant l'application.

7. Dispositif selon l'une des revendications 1 à 6, configuré de plus pour surveiller en ligne le résultat de la thérapie par une ou plusieurs sondes de mesure appropriées et pour tenir compte de leur signal ou de leurs signaux de mesure pour la commande du dispositif de dosage.

**ABBILDUNG 1**

**ABBILDUNG 2 (a)**

ABBILDUNG 2 (b)

ABBILDUNG 2 (c)

**ABBILDUNG 3**

**ABBILDUNG 4**

ABBILDUNG 5 (a)

ABBILDUNG 5 (b)

**ABBILDUNG 6 (a)**

**ABBILDUNG 6 (b)**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10160270
- DE 10345836
- DE 10345837
- US 2002173729 A
- US 5687208 A

**In der Beschreibung aufgeführte Nicht-Patentliteratur**

- Diprifusor™: Target Controlled Infusion (TCI) in anaesthetic practice. AstraZeneca Anaesthesia. 1998
- **S. WILLMANN ; J. LIPPERT ; M. SEVESTRE ; J. SOLODENKO ; F. FOIS ; W. SCHMITT.** PK-Sim®: a physiologically based pharmacokinetic 'whole-body' model. *Biosilico,* 2003, vol. 1, 121-124
- **P. S. PRICE ; R. B. CONOLLY ; C. F. CHAISSON ; E. A. GROSS ; J. S. YOUNG ; E. T. MATHIS ; D. R. TEDDER.** Modeling interindividual variation in physiological factors used in PBPK models of humans. *Crit. Rev. Toxicol.,* 2003, vol. 33, 469-503
- **A. OHNISHI.** A Review of Clinical Use of Theophylline in Acute Asthma: Factors Influencing Kinetic Disposition and Drug Interactions. *Methods Find. Exp. Clin. Pharmacol.,* 2000, vol. 22, 253-258
- **P. A. MITENKO ; R. I. OGILVIE.** Pharmacokinetics of intravenous Theophylline. *Clin. Pharm. Therap.,* 1973, vol. 14, 509-513
- **J. P. JAMESON ; A. MUNYIKA.** Theophylline Pharmacokinetics in Black Zimbabwean Males. *Therap. Drug Monitoring,* 1990, vol. 12, 54-58
- **P. GAL ; W. J. JUSKO ; A. M. YURCHAK ; B. A. FRANKLIN.** Theophylline disposition in obesity. *Clin. Pharmacol. Ther.,* 1978, vol. 23, 438-44
- **S. H. D. JACKSON ; A. JOHNSTON ; R. WOOLLARD ; P. TURNER.** The Relationship between Theophylline Clearance and Age in Adult Life. *Eur. J. Clin. Pharmacol.,* 1989, vol. 36, 29-34
- **Z. TRNAVSKA.** Theophylline Protein Binding. *Arzneim.-Forsch.,* 1990, vol. 40, 166-170
- **Z. KATO ; O. FUKUTOMI ; N. KONDO.** Developmental Changes of unbound Theophylline. *Ann Allergy Asthma Immunol.,* 1988, vol. 80, 517
- **Z. KATO ; O. FUKUTOMI ; N. KONDO.** Developmental Changes of unbound Theophylline. *Ann Allergy Asthma Immunol.,* 1998, vol. 80, 517
- **S. VALENTI ; P. CRIMI ; V. BRUSASCO.** Bioavailability and Pharmacokinetics of a New Controlled Release Theophylline Preparation in the Form of Capsules Containing Pellets. *Respiration,* 1987, vol. 52, 195-200
- **M.MÜLLER ; B. V. OSTEN ; R. SCHMID ; E. PIEGLER ; I. GERNGROSS ; H. BUCHEGGER ; H. G. EICHLER.** Theophylline kinetics in peripheral tissues in vivo in humans. *Naunyn-Schmiedeberg's Arch. Pharmacol.,* 1995, vol. 352, 438-441
- **M. C. MEYER ; E. J. JARVI ; A. B. STRAUGHN ; F. R: PELSOR ; R. L. WILLIAMS ; V. P. SHAH.** Bioequivalence of Immediate-release Theophylline Capsules. *Biopharm. Drug Dispos,* 1999, vol. 20, 417-419
- **P. A. MITENKO ; R I. OGILVIE.** Pharmacokinetics_ of intravenous Theophylline. *Clin. Pharm. Therap.,* 1973, vol. 14, 509-513